# EUROPEAN PATENT APPLICATION

(11) **EP 4 821 548 A2**
(43) Date of publication of application: **14.10.2026**
(21) Application number: 26202378.1
(22) Date of filing: 13.07.2022
(51) Int. Cl.: A61N 5/10

(54) **SCANNING DYNAMIC DEVICE FOR MINIBEAMS PRODUCTION**

(30) Priority: 05.08.2021 EP 21306092
(62) Document number(s) of the earlier application(s) in accordance with Art. 76 EPC: 22750710.0 / 4 380 682
(71) Applicant: Institut Curie, 75005 Paris (FR); Institut National de la Santé et de la Recherche Médicale, 75013 Paris (FR); Centre National de la Recherche Scientifique, 75016 Paris (FR); Université Paris-Saclay, 91190 Gif-sur-Yvette (FR)
(72) Inventor: SOTIROPOULOS, Marios, Rochester, Minnesota, 55902 (US); PREZADO, Yolanda, 91400 Orsay (FR)
(74) Representative: IPAZ

(57) **Abstract**

The invention relates to a scanning dynamic collimator device (SDD) for minibeam production. The SDD comprises a single slit collimator being mounted on a support. The SDD is arranged to translate the collimator in a plane perpendicular to a plane whereby the single slit extends and/or in a direction parallel to the plane whereby the single slit extends and/or to rotate the collimator relative to a rotation axis parallel to the plane whereby the single slit extends and to tilt the collimator relative to a predefined reference axis and/or relative to a point comprised in the single slit.

## Description

### Funding

The project leading to the results disclosed in the present invention has received funding from the European Research Council (ERC) under the European Union's Horizon 2020 research and innovation programme (grant agreement No 817908).

### Field of the invention

The present invention relates to minibeam radiation therapy and, in particular, with proton minibeam radiation therapy (pMBRT). Minibeam radiation therapy (MBRT) is a novel radiotherapy approach based on a strong dose modulation. An effect called "tissue sparing" can be significantly improved with the MBRT technique which requires to produce an array of minibeam. MBRT significantly increase dose tolerances and sparing of normal tissue. An array of minibeams consists in an alignment of parallel minibeams, each of the minibeam exhibits a width smaller than 1 mm full width at half maximum (FWHM) and two minibeams are usually separated by 2 to 4 mm. The tissue sparing effect is achieved if each beam of the minibeams array exhibits a size smaller than 1 mm FWHM. Such size cannot be obtained with conventional Pencil Beam Scanning nozzles (PBS). In a dose point of view, MBRT generates an array of areas of high dose values, called peaks, adjacent to areas of low dose values, called valleys. The shape and size of an array of minibeams is mainly characterized by the peak-to-valley dose ratio (PVDR).

Contrary to conventional proton therapy, the non-homogeneous dose deposition pattern of MBRT, alternation of regions of low and high dose, is a major advantage. MBRT usually provides homogeneous target dose coverage at the target while normal tissues located upstream benefit from the spatial fractionation of the dose. MBRT has already demonstrated a significant reduction of normal tissue toxicities as well as an equivalent or superior life.

The present invention deals with a scanning dynamic device (SDD) for minibeam and arrays of minibeams production.

### Background to the invention

Prior art discloses multislits collimator to produce minibeams by modulating a uniform field using a multislit collimator. Multislit collimators produce minibeams of fixed length and width. Such device requires to manufacture a new collimator each time a new minibeam array is required. Manufacture a multislits collimator is time consuming and expensive due to the required precision and accuracy in the manufacturing process.

It is also known to use pencil beam scanning to produce minibeams by scanning the target or area to be treated. The main issue of the PBS is that typical spot widths are around 5 to 10 mm FWHM which is too large to benefit from the sparing effect provided by the minibeams.

### Summary of the invention

An object of the invention is to provide:
- a SDD that allows generation of an array of minibeams of any shape and size, and/or
- a SDD that allows to modulate, in real time, the size and shape of a minibeam or of an array of minibeams, and/or
- a SDD that may be integrated directly on any beamline of a medical facility, in particular for radiotherapy treatment.

To this end, there is provided a scanning dynamic device (SDD) for minibeam production, the SDD comprising a single slit collimator being mounted on a support,
the SDD, preferably the support and/or the collimator, is arranged to:
- translate the collimator in a plane perpendicular to a plane whereby the single slit extends and/or in a direction parallel to, preferably comprised in, the plane whereby the single slit extends, and/or
- rotate the collimator relative to a rotation axis parallel to, preferably comprised in, the plane whereby the single slit extends, and
- tilt the collimator relative to a predefined reference axis and/or relative to a point comprised in the single slit.

Preferably, the SDD is arranged to produce or form or shape a proton minibeam. Preferably, the SDD is arranged to produce or shape or form an array of minibeam.

Preferably, the minibeam is produced from an incident beam. More preferably, the minibeam is produced from an incident beam in the form of a scanning beam.

Preferably, the SDD is arranged to adjust and/or modify the single slit position and/or angle so that the single slit extends in a plane parallel to, and preferably comprising, the incident beam.

The support and the collimator are mobile or movable and arranged to be moved so that the incident beam goes through the slit in a direction parallel to the plane whereby the single slit extends.

Preferably the plane whereby the single slit extends is parallel to, or comprised, a length of the single slit and/or perpendicular to a width of the single slit. More preferably, the plane whereby the single slit extends is parallel to, or comprised, the length of the single slit and comprises a median of the single slit, preferably the median connecting the opposed smallest sides of the single slit.

The predefined reference axis may be defined as the axis coinciding with the line extending along the center of the single slit when the SDD is off or in its reference position. The line extending along the center of the single slit may be defined as the central axis of the collimator which is:
- the axis comprising the points of intersections of the medians of the single slit and/or the points of intersections of the diagonals of the single slit, and/or
- the axis that corresponds to the line of intersection between the plane whereby the single slit extends which is parallel to, or comprised, the length of the single slit and, preferably, comprises a median of the single slit, more preferably the median connecting the opposed smallest sides of the single slit, and the plane perpendicular to the plane whereby the single slit extends which is parallel to, or comprised, the width of the single slit and comprises a median of the single slit, preferably the median connecting the opposed largest sides of the single slit.

Preferably, the SDD is arranged to adjust, in real time, its position and orientation, preferably by tilting and/or translating and/or rotating the collimator, to align the plane whereby the single slit extends with the scanning beam and/or, preferably, with the central axis of the collimator.

The collimator may be arranged to modulate a width of the single slit and/or a length of the single slit.

The collimator may exhibit a single slit with a fixed width and/or a fixed length. The collimator may be a single block in which the single slit, with fixed length and/or width, is managed.

The collimator may comprise two couple of parallel blocks arranged relative to each other to form the single slit.

Preferably, the blocks of one of the couple, or a direction in which a block of one of the couple extends or the largest size or dimension in which a block of one of the couple extends, is perpendicular to the blocks of the other couple.

Preferably, the blocks of a couple are parallel to each other.

A block may be a one-piece component or piece. Preferably, a block is not deformable.

Preferably, a distance between the two blocks of a couple may set or may define the width of the single slit and a distance between the two blocks of the other couple sets or defines the length of the single slit.

At least two blocks, preferably each of the blocks of a couple, more preferably each block of the collimator, being moveable, preferably relative to the support, in a direction perpendicular to the plane whereby the single slit extends and/or in a direction of a median connecting the opposed smallest sides of the single slit.

Preferably, one block is moveable relative to each of the other blocks and/or a block of a couple of blocks is moveable, more preferably requires to be moved, together with one of the blocks of the other couple of blocks.

Preferably, the SDD is arranged to adjust and/or modify the single slit position and/or angle so that the single slit extends in a plane parallel to an incident beam.

A couple of blocks, preferably each of the blocks of a couple, more preferably each block of the collimator, is moveable in the plane whereby the single slit extends and/or in the direction of a median connecting the opposed smallest sides of the single slit and the other couple of blocks is moveable in the direction of a median connecting the opposed smallest sides of the single slit and/or in the plane whereby the single slit extends.

Preferably, the at least two couple of blocks are movable by translation.

In a first configuration, the SDD may comprise at least two adjustable and/or movable overlapping areas or surfaces formed or defined or constituted or provided by the overlapping of the projection of one of the blocks of a couple of blocks onto each of the two blocks of the other couple, said projection being carried out according to a direction, said incident beam direction, comprised in the plane whereby the single slit extends and perpendicular to the median connecting the opposed smallest sides of the single slit or according to the direction extending from the downstream face towards the upstream face or conversely.

In the first configuration, when one block of each pairs of blocks are moved, the SDD may comprise three adjustable overlapping areas.

In the first configuration, the SDD may comprise four adjustable overlapping areas when the two blocks of one pair of blocks are moved, that is the two blocks of the same pair.

In the first configuration, the blocks of a couple are, preferably, moveable in the plane whereby the single slit extends and the other couple of blocks is moveable in the direction of a median connecting the opposed smallest sides of the single slit.

In a second configuration, the SDD may comprise at least two movable or mobile contact areas, each movable contact area is located between, or comprises or is formed by or is constituted of, a surface of a block of a couple in contact with a surface of a block of the other couple.

In the second configuration, the blocks of each couples are, preferably, moveable in the plane whereby the single slit extends and in the direction of a median connecting the opposed smallest sides of the single slit.

Unless specified, the features described in the present application apply to the first and second configuration.

Preferably, the at least two movable contact areas and/or the at least two adjustable overlapping areas and/or the at least two movable blocks being arranged to:
- modulate the width of the single slit and/or the length of the single slit, and/or
- translate the single slit, relative to the support, in a plane perpendicular to the plane whereby the single slit extends.

The at least two movable contact areas and/or the at least two adjustable overlapping areas may each be comprised on a face, or a contact face of a block. A surface area of the at least two movable contact areas and/or the at least two adjustable overlapping areas may each be modulated by translation of one block alongside a face, or a contact face, of another block.

In the first configuration, each block of a couple may comprises a face, preferably one face and more preferably a single face, that:
- faces, or that is in front of or opposite to, one face of each of the two blocks of the other couple,
- comprises two adjustable overlapping surfaces, each of the two adjustable overlapping surfaces faces one adjustable overlapping surfaces of one block of the other couple.

The two adjustable surfaces of a block may correspond to or be identical to or coincide with the at least two adjustable overlapping areas.

Preferably, an adjustable overlapping surface, preferably each of the two adjustable overlapping surfaces, faces one face of each of the two blocks of the other couple. More preferably, an adjustable overlapping surface, preferably each of the two adjustable overlapping surfaces, faces one adjustable overlapping surface of a block of the other couple of block.

In the second configuration, each block of a couple may have two contact faces, a surface of one of the two contact faces is in contact with a surface of a contact face of a block of the other couple and a surface of the other of the two contact faces is in contact with a surface of a contact face of the other block of the other couple.

A contact face or an adjustable overlapping surface may be defined as all or part of a surface of a face of a block.

Preferably, in the present application, any feature related to the movable contact areas applies to the contact faces and/or to the surfaces of the blocks in contact and vice versa.

Preferably, in the present application, any feature related to the adjustable overlapping areas applies to the adjustable overlapping surfaces and vice versa.

A movable contact area may comprise, preferably consists in, two surfaces, or contact surfaces, of two blocks, each, of a different couple of parallel blocks. Preferably, a movable contact area comprises, preferably consists in, a surface, or contact surface, of a contact face of a block of a considered couple of parallel blocks and a surface, or contact surface, of a contact face of a block of the other couple of parallel blocks.

An adjustable overlapping areas may comprise, preferably consists in, a surface, or in an adjustable overlapping surface, of one block. An adjustable overlapping areas may be comprised, preferably consists in, a surface of a block. An adjustable overlapping areas may be comprised in a plane parallel to surface of a block and/or in a plane perpendicular to the plane whereby the single slit extends. An adjustable overlapping areas may be comprised in a plane located between a surface of a block a considered couple, preferably between one surface of a first block of a considered couple and one surface of the other block of the considered couple which is comprised in the same plane as the surface of the first block of the considered couple, and a surface of a block of the other couple that faces the considered couple of blocks, preferably one surface of a first block of the other couple that faces the considered couple of blocks and one surface of the other block of the other couple that faces the considered couple of blocks; said surface the other block of the other couple that faces the considered couple of blocks being comprised in the same plane as the surface of the first block of the other couple that faces the considered couple of blocks.

Preferably, the at least two blocks are moveable, in a direction perpendicular to the plane whereby the single slit extends and/or in a direction of a median connecting the opposed smallest sides of the single slit, so that the at least two movable contact areas, preferably each of the movable contact areas, being each translated alongside a contact face of a different block.

In the first configuration of the SDD, the collimator may comprise four adjustable overlapping areas, the four movable contact areas of the collimator forms two couples of parallel adjustable overlapping areas, the two adjustable overlapping areas of a couple move together or jointly. Preferably, the four movable contact areas of the collimator are comprised in parallel planes or in a same and single plane.

In the second configuration of the SDD, the collimator may comprise four movable contact areas, the four movable contact areas are each comprised in a different plane and are parallel two by two thus forming two couples of parallel movable contact areas.

In the first configuration, a couple of parallel blocks, said first couple, may be arranged on top of the other couple, said second couple; the single slit may extend from a face, or a surface of the face, said downstream or distal face, of the collimator formed by the faces of the blocks of the first couple located on a downstream side of the collimator to a face, said upstream or proximal face, of the collimator formed by the faces of the blocks of the second couple located on an upstream side of the collimator.

In the second configuration, each block of a couple may be arranged alongside with the two blocks of the other couple; the single slit may extend from a face, or a surface of the face, said downstream face, of the collimator formed by the faces of each blocks located on a downstream side of the collimator to a face, said upstream face, of the collimator formed by the faces of each blocks located on an upstream side of the collimator.

Preferably, the downstream and/or the upstream face extend essentially along a plane.

The terms "downstream" and "upstream" may be defined relative to the direction in which the incident beam propagates or the direction extending between the SDD and a target.

Preferably, the SDD is arranged to tilt the collimator relative to a point comprised:
- in the plane whereby the single slit extends, and/or
- in a plane, said median radial plane, equidistant from the upstream face and the downstream face of the collimator and perpendicular to the plane whereby the single slit extends. More preferably, the SDD is arranged to tilt the collimator relative to a point of intersection between the plane whereby the single slit extends and the median radial plane.

In the first configuration, the four adjustable overlapping areas may be comprised in a plane perpendicular to the plane whereby the single slit extends and/or may be located between the first and the second couple of blocks.

In the second configuration, the four movable contact areas may be comprised in planes extending from the downstream face to the upstream face of the collimator, one movable contact area of a considered block of a considered couple of parallel blocks may be:
- parallel to one movable contact area of a block of the other couple of parallel blocks, parallel to one movable contact area of the other block of the other couple of parallel blocks and parallel to one movable contact area of the other block of the considered couple,
- perpendicular to the other movable contact area of the block of the other couple of parallel blocks, perpendicular to the other movable contact area of the other block of the other couple of parallel blocks, perpendicular to the other movable contact area of the other block of the considered couple and/or perpendicular to the other movable contact area of the considered block of the considered couple.

Preferably, in the first configuration, the four movable areas are parallel to each other.

Preferably, in the second configuration, each of the contact faces are perpendicular to the upstream and the downstream faces.

Preferably, in the second configuration, the two contact faces of a block of a considered couple of parallel blocks are each parallel to one of the two contact faces of the other block of the considered couple of parallel blocks.

The support may comprise:
- a base comprising a through hole through which, or by which, the incident beam is intended to propagate towards, and preferably through, the collimator, and
- a plate on which the collimator is mounted, the plate comprising a through hole through which the incident beam is intended to propagate from the base towards the collimator; the plate being arranged to be rotated, translated and tilt relative to the base.

The support may be a hexapod.

The device may also comprise, or intended to be coupled or connected with, control means to control and/or command the SDD. The control means may be arranged to control the translation and/or the rotation and/or the tilting of the collimator and/or to modulate the width and/or the length of the single slit and/or to control the motion of the blocks of the collimator and/or to control the motion of the support, or preferably the motion of the plate relative to the base.

According to the invention, it is also provided a use of the SDD for Minibeam Radiation Therapy (MBRT). Preferably, a use of the SDD for proton Minibeam Radiation Therapy (pMBRT).

There is also provided a method for producing a minibeam comprising the steps of:
- providing an incident beam in the form of a scanning beam, and
- moving a single slit of a collimator, the step consisting of moving the single slit of the collimator comprises:
   - translating a collimator, by means of a scanning dynamic device (SDD), in a plane perpendicular to a plane whereby the single slit extends and/or in a direction parallel to the plane whereby the single slit extends so that, at least temporarily and at least in part, preferably temporarily and in part only, the scanning beam goes through the single slit, and/or
   - rotating the collimator, by means of the SDD, relative to a rotation axis parallel to the plane whereby the single slit extends and
   - tilting the collimator, by means of the SDD, relative to a predefined reference axis and/or relative to a point comprised in the single slit so that the scanning beam remains parallel to a plane whereby the single slit extends and/or to compensate the incident beam deflection.

Preferably, the incident beam is intended to go through the single slit to produce the minibeam.

The step consisting of moving the single slit is carried out so that the incident beam after going through the single slit produces the minibeam or the array of minibeams.

Preferably, the scan of the scanning beam is carried out according to a predetermined pattern. Preferably, the predetermined pattern is parallel lines.

Preferably, the scanning beam may be a Pencil Beam Scanning (PBS).

Preferably the step of moving the single slit is carried out or implemented by the SDD according to the invention, more preferably by means of the support on which the collimator is mounted.

The step of moving the collimator may produce one minibeam, that is one high energy line or peak or one high dose line or peak. Preferably, the step of moving the collimator may produce an array of minibeams, that is an array of high dose values or peaks adjacent to areas of low dose values or valleys.

In a first aspect of the method, the step of moving the collimator may comprise the steps of:
- positioning the single slit so that a central plane of the single slit which extends along the plane whereby the single slit extends and comprises a length of the single slit is aligned with a scan direction of the scanning beam,
- when the scanning beam reaches a center of the single slit, translating the single slit according to the scan direction so that the scanning beam remains at the center of the single slit.

Preferably, the step of moving the collimator further comprises the steps consisting of:
- after positioning the single slit, emitting the scanning beam and moving the scanning beam according to the scan direction, and/or
- stopping the movement of the single slit according to the scan direction and, preferably when the scanning beam reaches a longitudinal end of the single slit, stop the scanning beam emission.

Preferably, the first aspect of the method is implemented or carried out by the second configuration of the SDD according to the invention.

In a second aspect of the method, the step of moving the collimator comprises the steps of:
- positioning the single slit so that the scanning beam, when emission of the scanning beam is started, goes through the single slit at a longitudinal or lengthwise end of the single slit,
- positioning the single slit so that a non-zero angle is formed between a length of the single slit and a scan direction of the scanning beam,
- emitting, or starting the emission of, the scanning beam and moves the scanning beam according to the scan direction,
- translating the single slit in a direction perpendicular to the plane whereby the single slit extends so that the scanning beam goes through the single slit,
- when the scanning beam reaches the other longitudinal end of the single slit, stopping the scanning beam emission.

Preferably, the second aspect of the method is implemented or carried out by the first configuration of the SDD according to the invention.

Preferably, the non-zero angle is formed between the central plane of the single slit, which extends along the plane whereby the single slit extends and comprises the length of the single slit, and a scan direction of the scanning beam.

In the first and second aspect of method, the step of moving the collimator produces one minibeam, that is one high energy line or peak or one high dose line or peak. To produce an array of minibeam or an array of high dose values or peaks adjacent to areas of low dose values or valleys, the step of moving the collimator according to the first and second aspect of method may be reiterated, preferably several times.

The method may comprise, when implemented or carried out by the SDD according to the second configuration, the translation of a block of a considered couple relative to a block of the other couple results in the translation of the other block of the other couple relative to the other block of the considered couple.

The method may comprise, when implemented or carried out by the SDD according to the first configuration, the translation of a block of a considered couple relative to a block of the other couple results in the translation of the block of the considered couple relative to the other block of the other couple.

The method according to the invention may be implemented by any scanning collimator. Preferably, the SDD according to the invention is arranged, more preferably specifically arranged, to implement the method according to the invention. Thus, any feature of the SDD may be transpose and/ integrate in the method according to the invention and vice versa.

### Brief description of the drawings

Further inventive objects, features and advantages will become apparent from the following detailed description of several embodiments of the invention with references to the drawings, in which:
- FIGURE 1 is a schematic view of a scanning beam collimator device according to the invention (SDD),
- FIGURES 2a and 2b are schematic side views of two embodiments of SDDs,
- FIGURES 2c and 2d are schematic top views of the two embodiments of SDDs illustrated on FIGURES 2a and 2b,
- FIGURE 3a, 3b, 3c and 3d show schematic representations of the scanning beam 15, the collimator 2 and a target 18,
- FIGURES 4 and 5 are schematic views of two embodiments of the method for producing a minibeam according to the invention.

### Detailed description of embodiments of the invention

The embodiments hereinafter described are not restrictive; other embodiments comprising a selection of features described hereinafter may be considered. A selection may comprise features isolated from a set of features (even if this selection is isolated among a sentence comprising other features thereof), if the selection is sufficient to confer a technical advantage or to distinguish the invention form the state of the art. This selection comprises at least a feature, preferably described by its technical function without structural features, or with a part of structural details if this part is sufficient to confer a technical advantage or to distinguish the invention form the state of the art on its own.

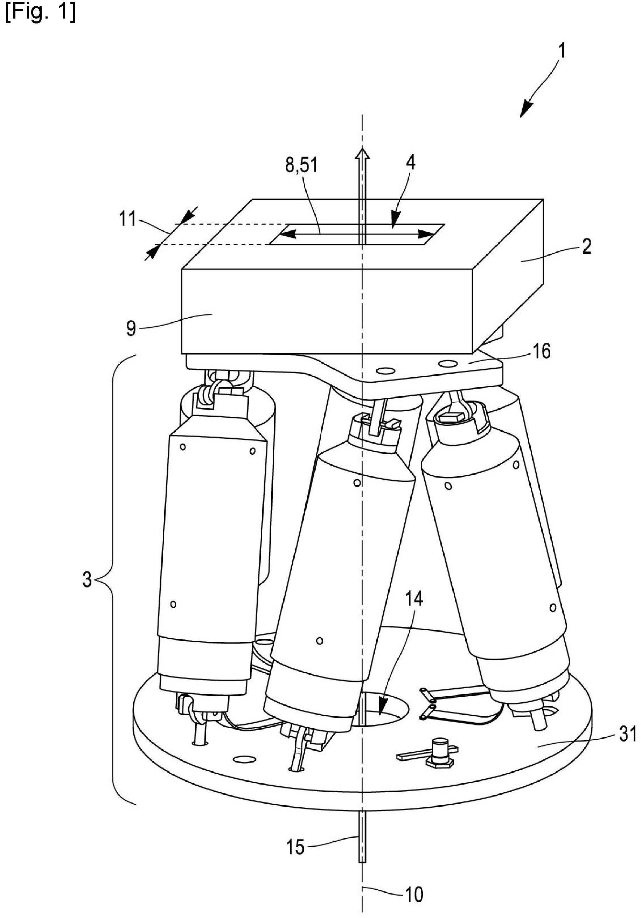

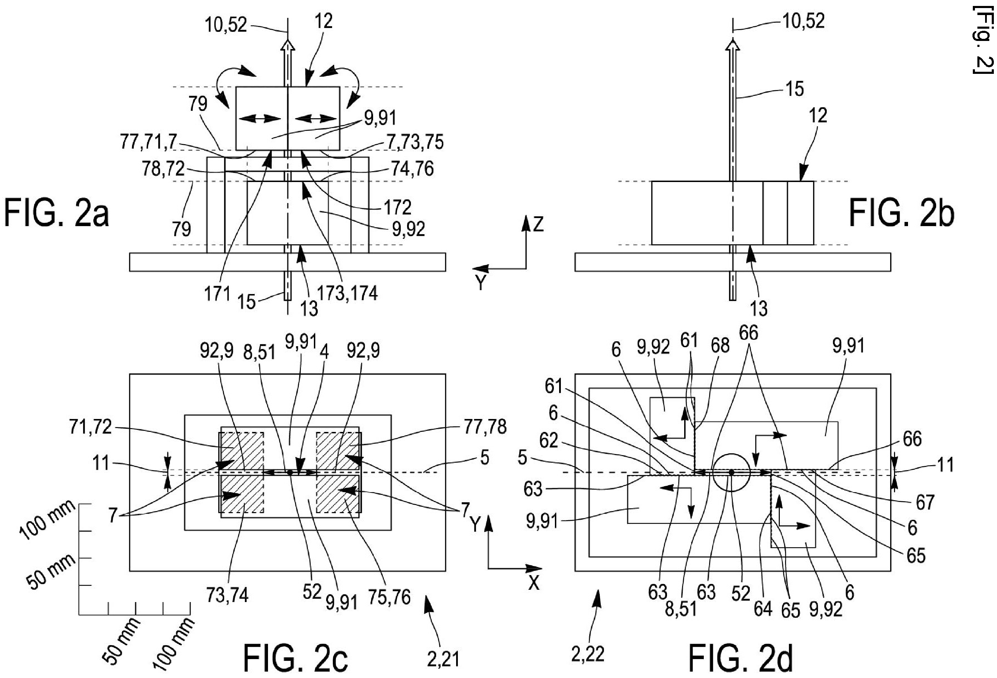

In reference to FIGURES 1 and 2, an embodiment of a scanning dynamic collimator device (SDD) 1 according to the invention is described. This SDD 1 is intended to be used for minibeam radiation therapy and in particular for proton Minibeam Radiation Therapy (pMBRT). The SDD 1 for minibeam production comprises a single slit collimator 2 being mounted on a support 3. According to FIGURE 1, the single slit collimator 2 is monobloc or one-piece. In that case, the slit 4 has fixed width 11 and length 8. The SDD 1, and the support 3 according to the present embodiment, are arranged to translate the collimator 2 in a plane XY perpendicular to a plane XZ whereby the single slit 4 extends. The SDD 1, and the support 3 according to the present embodiment, may also be arranged to translate the collimator 2 and in a direction Z parallel to the plane XZ whereby the single slit 4 extends. In other words, in this embodiment, the SDD 1, and in particular the support 3, are arranged to translate the single slit 4 according to direction X, and to the opposite direction, and/or according to Y, and to the opposite direction, and/or according to Y, and to the opposite direction. The SDD 1, and the support 3 according to the present embodiment, are arranged to rotate the collimator 2 relative to a rotation axis parallel to the plane XZ whereby the single slit 4 extends. The SDD 1, and the support 3 according to the present embodiment, are arranged to tilt the collimator 2 relative to a predefined reference axis 10.

In reference to FIGURE 2, the collimator 2 comprises several blocks 9. The collimator 2 comprises two couple 91, 92 of parallel blocks 9 arranged relative to each other to form the single slit 4. The distance between the two blocks 9 of a couple 91 sets the width 11 of the single slit 4 and a distance between the two blocks 9 of the other couple 92 sets the length 8 of the single slit 4. In other words, in this embodiment the SDD 1, and in particular the support 3 and preferably the at least two movable blocks 9, are arranged to translate the single slit 4 according to direction X, and to the opposite direction, and/or according to Y, and to the opposite direction, and/or according to Y, and to the opposite direction.

In a preferred embodiment, and in reference to FIGURE 2, the collimator 2 is arranged to modulate a width 11 of the single slit 4 and a length 8 of the single slit 4. The collimator 2 comprises at least two blocks 9, four according to the embodiment, being moveable in the direction Y perpendicular to the plane XZ whereby the single slit 4 extends and in the direction X parallel to the median 51 connecting the opposed smallest sides of the single slit 4.

According to the embodiment, the support 3 comprises:
- a base 31 comprising a through hole 14 through which the incident beam 15 is intended to propagate towards the collimator 2, and
- a plate 16 on which the collimator 2 is mounted. The plate 16 comprises a through hole through which the incident beam 15 is intended to propagate from the base 31 towards the collimator 2. The plate 16 is arranged to be rotated, translated and tilt relative to the base 31.

According to the embodiment, the support 3 is a hexapod.

In reference to FIGURES 2a and 2c, it is illustrated a first configuration 21 of the SDD 1 with modular width 11 and length 8. In this first configuration 21, the collimator 2 comprises at least two adjustable overlapping areas 7 formed by the overlapping of the projection of one of the blocks 9 of the couple 91 of blocks onto each of the two blocks 9 of the other couple 92. The projection is carried out according to the direction opposite to direction Z which is comprised in the plane XZ whereby the single slit 4 extends. Direction Z, and the opposite direction, being perpendicular to the median 51 connecting the opposed smallest sides of the single slit 4. The least two adjustable overlapping areas 7, by means of the at least two movable blocks 9 motion, are arranged to modulate the width 11 of the single slit 4 and the length 8 of the single slit 4. Modulate the surfaces of the two adjustable overlapping areas 7, by means of the at least two movable blocks 9 motion, modulate the width 11 of the single slit 4 and the length 8 of the single slit 4. The least two adjustable overlapping areas 7, by means of the at least two movable blocks 9 motion, are also arranged to translate the single slit 4, relative to the support 3, in a plane XY perpendicular to the plane XZ whereby the single slit extends.

The projection according to the embodiment is chosen arbitrarily, projection of blocks 9 of the couple 92 onto the blocks 9 of the other couple 91 should have been chosen.

According to the embodiment, the fourth blocks 9 are movable, so that the collimator 2 comprises four adjustable overlapping areas 7 formed by the overlapping of the projection of the two blocks 9 of the couple 91 onto the two blocks 9 of the other couple 92.

According to the first configuration 21, each block 9 of the couples 91, 92 comprises one face 171, 172, 173, 174 facing one face 174, 173, 172, 171 of each of the two blocks 9 of the other couple 92, 91. The faces 171 and 172 of the couple 91 face the faces 173 and 174 of the couple 92 and conversely. Each block 9 of the couples 91, 92 also comprises two adjustable overlapping surfaces 71, 72, 73, 74, 75, 76, 77, 78. Each of the two adjustable overlapping surfaces 71, 72, 73, 74, 75, 76, 77, 78 faces one adjustable overlapping surfaces of one block 9 of the other couple. Face 171 comprises the two adjustable overlapping surfaces 71 and 77 that face adjustable overlapping surface 72 of face 173 and adjustable overlapping surface 78 of face 174. Face 172 comprises the two adjustable overlapping surfaces 73 and 75 that face adjustable overlapping surface 74 of face 173 and adjustable overlapping surface 76 of face 174.

According to the first configuration 21, the collimator 2 comprises four adjustable overlapping areas 7. The four adjustable overlapping areas 7 of the collimator 2 are located in a same and single plane 79 which is comprised or located, by convention or choice, between the two parallel faces 171, 172 of blocks 9 of couple 91 and the two parallel surfaces 173, 174 of blocks 9 of the other couple 92 that face the couple 91. The four adjustable overlapping areas 7 of the collimator 2 form two couples of parallel adjustable overlapping areas 7. The two adjustable overlapping areas 7 of a couple 91, 92 move together when one block 9 of a couple 91, 92 is translated.

According to the first configuration 21, a couple 91 of parallel blocks 9, said first couple 91, is arranged on top of the other couple 92, said second couple 92. The single slit 4 extends from a face 12, said downstream face 12, of the collimator 2 formed by the faces of the blocks 9 of the first couple 91 located on a downstream side of the collimator 2 to a face 13, said upstream face 13, of the collimator 2 formed by the faces of the blocks 9 of the second couple 92 located on an upstream side of the collimator 2.

As for the projection, the choice of the plane 79 is arbitrary. The plane 79 is perpendicular to the plane XZ whereby the single slit 4 extends. The plane 79 is located between the first couple 91 and the second couple 92. The plane 79 is located between the parallel faces 171, 172 of the blocks 9 of the couple 91 and the parallel faces 173, 174 of the blocks 9 of the couple 92.

In reference to FIGURES 2b and 2d, it is illustrated a second configuration 22 of SDD 1 with modular width 11 and length 8. In this second configuration 22, the collimator 2 comprises at least two movable contact areas 6. Each movable contact area 6 is located between a surface of a block 9 of a couple 91, 92 in contact with a surface of a block 9 of the other couple 92, 91 and conversely. The at least two movable contact areas 6 are arranged to modulate the width 11 of the single slit 4 and the length 8 of the single slit 4. The at least two movable contact areas 6 are arranged to translate the single slit 4, relative to the support 3, in a plane XY perpendicular to the plane XZ whereby the single slit extends and to the plane YZ. In other words, the at least two movable contact areas 6 are arranged to translate the single slit 4 according to direction X, and the opposite direction, and/or according to Y, and the opposite direction.

In the second configuration 22, the collimator 2 comprises four movable contact areas 6, the four movable contact areas 6 are each comprised in a different plane and are parallel two by two thus forming two couples of parallel movable contact areas 6. Two movable contact areas 6 are comprised in a plane parallel to the plane XZ and two are movable contact areas 6 comprised in a plane parallel to the plane YZ.

In the second configuration 22, each block 9 of a couple 91, 92 has two contact faces 61, 62, 63, 64, 65, 66, 67, 68. The surface of one of the two contact faces 61, 62, 63, 64, 65, 66, 67, 68 of a block 9 of one of the couple 91, 92 is in contact with a surface of a contact face 61, 62, 63, 64, 65, 66, 67, 68 of a block 9 of the other couple 91, 92 and the surface of the other face of the two contact faces 61, 62, 63, 64, 65, 66, 67, 68 is in contact with a surface of a contact face 61, 62, 63, 64, 65, 66, 67, 68 of the other block 9 of the other couple 91, 92. The contact face 61 of the block 9 of couple 92 is in contact with the contact face 68 of the block 9 of the couple 91 and the contact face 62 of the block 9 of couple 92 is in contact with the contact face 63 of the block 9 of the couple 91. The contact face 64 of the block 9 of couple 91 is in contact with the contact face 65 of the block 9 of the couple 92 and the contact face 66 of the block 9 of couple 91 is in contact with the contact face 67 of the block 9 of the couple 92.

In the second configuration 22, each block 9 of a couple 91, 92 is arranged alongside with the two blocks 9 of the other couple 92, 91. The single slit 4 extends from a face 12, said downstream face 12, of the collimator 2 formed by the faces of the four blocks 9 located on a downstream side of the collimator 2 to a face 13, said upstream face 13, of the collimator 2 formed by the faces of the four blocks 9 located on an upstream side of the collimator 2.

In the second configuration 22, the four movable contact areas 6 are comprised in planes XZ et YZ extending from the downstream face 12 to the upstream face 13 of the collimator 2.

One movable contact area 6 of a considered block 9 of a considered couple 91, 92 of parallel blocks is:
- parallel to one movable contact area 6 of a block 9 of the other couple 91, 92 of parallel blocks 9,
- parallel to one movable contact area 6 of the other block 9 of the other couple 91, 92 of parallel blocks 9, and
- parallel to one movable contact area 6 of the other block 9 of the considered couple 91, 92,
- perpendicular to the other movable contact area 6 of the block 9 of the other couple 91, 92 of parallel blocks,
- perpendicular to the other movable contact area 6 of the other block 9 of the other couple 91, 92 of parallel blocks, and
- perpendicular to the other movable contact area 6 of the other block 9 of the considered couple 91, 92,
- perpendicular to the other movable contact area 6 of the considered block 9 of the considered couple 92.

In other words, the movable contact area 61 of the block 9 of the couple 92 is:
- parallel to the movable contact area 68 of the block 9 of the other couple 91,
- parallel to the movable contact area 64 of the other block 9 of the other couple 91, and
- parallel to the movable contact area 65 of the other block 9 of the considered couple 92,
- perpendicular to the other movable contact area 66 of the block 9 of the other couple 91,
- perpendicular to the other movable contact area 67 of the other block 9 of the other couple 91 of parallel blocks, and
- perpendicular to the other movable contact area 63 of the other block 9 of the considered couple 92,
- perpendicular to the other movable contact area 62 of the same block 9 of the same couple 92.

It is also described an embodiment of a method for producing a minibeam for radiation therapy. In particular, a proton minibeam radiation therapy (pMBRT). The method comprises the steps of:
- providing an incident beam 15 in the form of a scanning beam 15, and
- moving the single slit 4 of a collimator 2. The method according to the invention is implemented by a scanning collimator. The SDD 1 according to the invention is particularly suitable to implement the method according to the invention. The scanning beam 15 is a pencil beam scanning (PBS) 15 according to the embodiment.

The step consisting of moving the single slit 4 of the collimator 2 comprises the step of translating the collimator 2, by means of the SDD 1, in the plane XY perpendicular to a plane XZ whereby the single slit 4 extends and in a direction parallel to the plane XZ whereby the single slit 4 extends. The step consisting of moving the single slit 4 allows the scanning beam 15, when moving, to goes through the collimator 2 via the single slit 4 at least temporarily and at least in part. The step consisting of moving the single slit 4 of the collimator 2 also comprises rotating the collimator 2, by means of the SDD 1, relative to a rotation axis parallel to the plane XZ whereby the single slit 4 extends. The step consisting of moving the single slit 4 of the collimator 2 also comprises tilting the collimator 2, by means of the SDD 1, relative to the predefined reference axis 10 and/or relative to a point comprised in the single slit 4 so that the scanning beam 15 remains parallel, constantly, to a plane XZ whereby the single slit 4 extends.

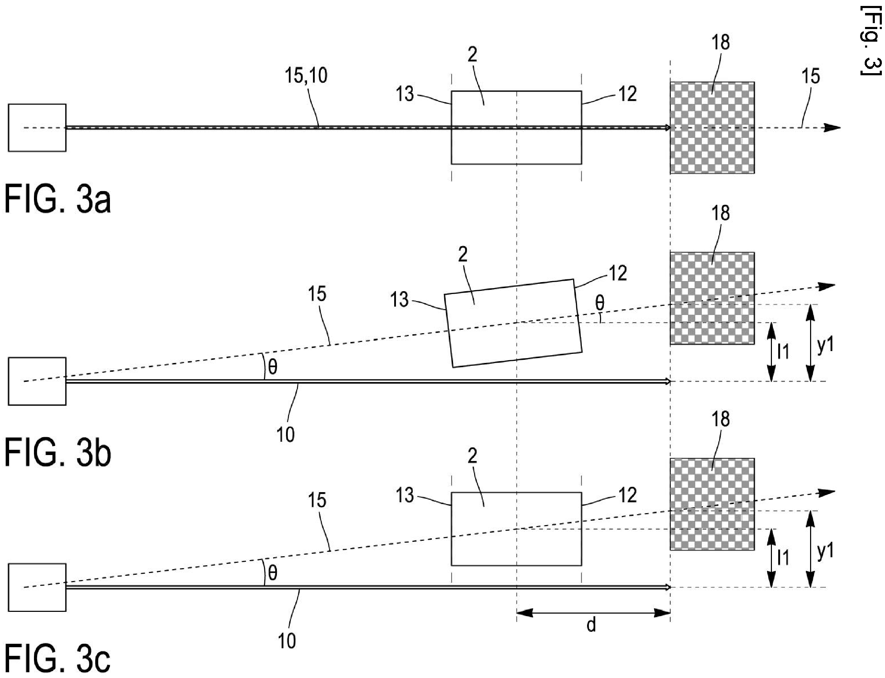

In reference to FIGURE 3, it is illustrated the schematic representation of the scanning beam 15, the collimator 2 and a target 18. FIGURE 3A) shows the incident beam 15 propagating according to the predefined reference axis 10. When the scanning beam 15 is off or in its reference position, namely coinciding with the predefined reference axis 10, the SDD 1 is positioned and oriented so that the predefined reference axis 10 coincide with the central axis 52 of the collimator 2 extending along the center of the single slit 4. The central axis 52 of the single slit 4 is comprised in the central plane 5 of the single slit 4.

In reference to FIGURES 3B) and 3C), when the scanning beam 15 scans the target 18, the scanning beam 15 moves and forms an angle θ with the predefined reference axis 10. On FIGURE 3C), the translation of the collimator 2 in the plane XY without tilting the collimator 2 is illustrated. In that case, the scanning beam 15 impinges the walls of the blocks 9 defining or delimiting the single slit 4 so that the scanning beam 15 get through only partially or does not get through the collimator 2. To remedy this issue, as illustrated FIGURE 3B), the collimator 2 is tilted, in real time, in addition to be translated when the scanning beam 15 moves so that the scanning beam 15 coincide with the central axis 52 of the collimator 2 at any moment.

In order for the central axis 52 of the collimator 2 to coincide at any moment with the scanning beam 15, the method may also comprise the adjustment and/or control of the orientation and/or position of the collimator 2. In other words, the method may also comprise the tilting and/or the translation and/or the rotation of the collimator 2, relative to the scanning beam 15 and/or to the predefined reference axis 10. The control is carried out, in real time, by a control means, a command unit for example, that commands and or controls the motions (translation, tilt and rotation) of the SDD 1, that is the collimator 2 and/or the blocks 9.

The method may also comprise the step of monitoring the alignment of the central axis 52 of the collimator 2 with the scanning beam 15. The monitoring may be implement by the command unit based on measurement data of the scanning beam 15 downstream the collimator 2. The data may be acquired by a sensor, position and/or dose sensor, positioned between the downstream face 12 of the collimator 2 and the target 18.

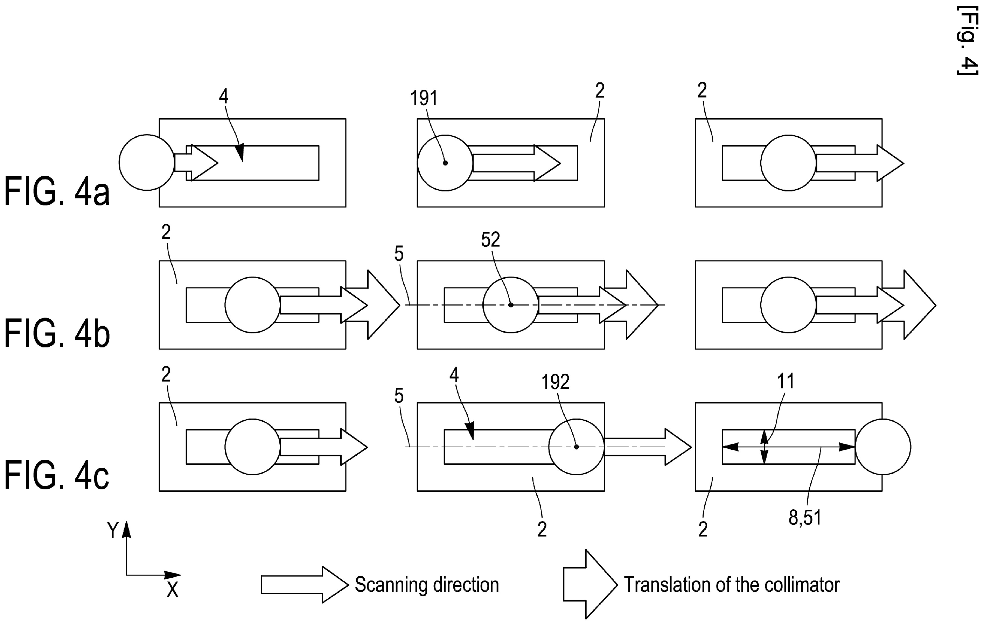

In a first embodiment of the method illustrated FIGURE 4, and before the scanning beam 15 is on, the step of moving the collimator 2 also comprises the step of positioning the single slit 4 so that a central plane 5 of the single slit 4, which extends along the plane XZ whereby the single slit 4 extends and comprises a length 8 of the single slit 4, is aligned with a scan direction of the scanning beam 15, that is the scan direction is comprised in the central plane 5 of the slit 4. When the scanning beam 15 reaches the center 52 of the single slit 4, as illustrated FIGURE 4A), the step of moving the collimator 2 comprises the step of translating the single slit 4, preferably the collimator 2, according to the scan direction so that the scanning beam 15 remains at the center 52 of the single slit 4 during the scanning, as illustrated FIGURE 4B) and so that the scanning beam 15 coincide with the central axis 52 of the collimator 2 at any moment. As illustrated FIGURE 4C), when a desired line constituting one minibeam of an array of minibeams has been carried out, the translation of the collimator 2 according to the scan direction is stopped and the scanning beam 15 still scans the target 18 until the edge of the collimator 2 is reached, then the scanning beam 15 is turned off.

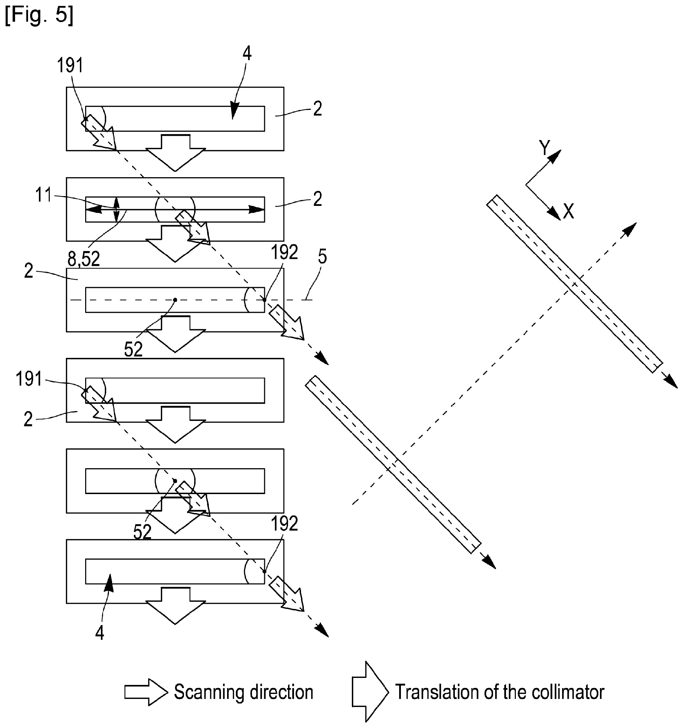

In a second embodiment of the method illustrated FIGURE 5, the step of moving the collimator 2 comprises also the steps of:
- positioning the single slit 4 so that the scanning beam 15, when emission of the scanning beam 15 is started, goes through the single slit 4 at a longitudinal end 191 of the single slit 4 and so the scanning beam 15 is comprised in the central plane 5 of the slit 4,
- positioning the single slit 4 so that a non-zero angle is formed between the length 8 of the single slit 4 and a scan direction of the scanning beam 15,
- turn on the scanning beam 15 and moves the scanning beam 15 according to the scan direction,
- translating the single slit 4 in a direction perpendicular to the plane XZ whereby the single slit 4 extends so that the scanning beam 15 goes through the single slit 4 and so the scanning beam 15 is comprised in the central plane 5 of the slit 4 at any moment,
- when the scanning beam reaches the other longitudinal end 192 of the single slit 4, turn off the scanning beam 15 emission.

Afterwards, the scanning beam 15, when turn off, and the single slit 4 and/or the collimator 2 are moved and positioned so that so that the scanning beam 15, when emission of the scanning beam 15 is started again, goes through the single slit 4 at a longitudinal end 191 of the single slit 4 and so the scanning beam 15 is comprised in the central plane 5 of the slit 4. In particular, the scanning beam 15 and the single slit 4 and/or the collimator 2 are positioned so that so that the longitudinal end 191 of the single slit 4 coincide with the end or extremity of a line constituting one minibeam of an array of minibeams.

The first and the second embodiments of the method may be implement independently, are not mutually exclusive and may be combined.

In some embodiment, the MSN according to the invention may be used in the treatment of cancer including but are not limited to solid tumors such as skin carcinomas, breast carcinomas, brain carcinomas, cervical carcinomas, testicular carcinomas rectum carcinoma, anal carcinoma, cardiac sarcoma (angiosarcoma, fibrosarcoma, rhabdomyosarcoma, liposarcoma), myx-oma, rhabdomyoma, fibroma, lipoma and teratoma; lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma, gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, Villous adenoma, hamartoma, leiomyoma); Genitourinary tract: kidney (adenocarcinoma, Wil-m's tumor nephroblastoma, lymphoma, leukemia), bladder and urethra (Squamous cell carcinoma, transitional cell carcinoma, adenocarcinoma), prostate (adenocarcinoma, Sarcoma), testis (seminoma, teratoma, embryonal carcinoma, teratocarcinoma, choriocarcinoma, Sarcoma, interstitial cell carcinoma, fibroma, fibroadenoma, adenomatoid tumors, lipoma); Liver: hepatoma (hepatocellular carcinoma), cholangiocarcinoma, hepatoblastom, angiosarcoma, hepatocellular adenoma, hemangioma; Bone: osteogenic sarcoma (osteosarcoma), fibrosarcoma, malignant fibrous histiocytoma, chon-drosarcoma, Ewing's sarcoma, malignant lymphoma (reticulum cell Sarcoma), multiple myeloma, malignant giant cell tumor chordoma, osteochron-froma (osteocartilaginous exostoses), benign chondroma, chondroblastoma, chondromyxofibroma, osteoid osteoma and giant cell tumors, Nervous System: skull (osteoma, hemangioma, granuloma, Xanthoma, Osteitis defor-mans), meninges (meningioma, meningiosarcoma, gliomatosis), brain (as-trocytoma, medulloblastoma, glioma, ependymoma, germinomapinealoma, glioblastoma multiform, oligodendroglioma, Schwannoma, retinoblastoma, congenital tumors), Spinal cord (neurofibroma, meningioma, glioma, Sarcoma); Gynecological: uterus (endometrial carcinoma), cervix (cervical carcinoma, pre-tumor cervical dysplasia), ovaries (ovarian carcinoma, Serous cystadenocarcinoma, mucinous cystadenocarcinoma, unclassified carcinoma, granulosa-thecal cell tumors, Sertoli-Leydig cell tumors, dysgerminoma, malignant teratoma), Vulva (Squamous cell carcinoma, intraepithelial carcinoma, adenocarcinoma, fibrosarcoma, melanoma), vagina (clear cell carcinoma, Squamous cell carcinoma, botryoid Sarcoma embryonal rhabdomyosarcoma, fallopian tubes (carcinoma); Skin: malignant melanoma, basal cell carcinoma, Squamous cell carcinoma, Karposi's Sarcoma, moles dysplastic nevi, lipoma, angioma, dermatofibroma, keloids, psoriasis, and Adrenal glands: neuroblastoma.

As used herein, the term "cancer" refers to any cancer that may affect any one of the following tissues or organs: breast; liver; kidney; heart, me-diastinum, pleura; floor of mouth; lip; salivary glands; tongue; gums; oral cavity; palate; tonsil; larynx; trachea; bronchus, lung; pharynx, hypopharynx, oropharynx, nasopharynx; esophagus; digestive organs such as stomach, intrahepatic bile ducts, biliary tract, pancreas, small intestine, colon; rectum; urinary organs such as bladder, gallbladder, ureter; rectosigmoid junction; anus, anal canal; skin; bone; joints, articular cartilage of limbs; eye and adnexa; brain; peripheral nerves, autonomic nervous system; spinal cord, cranial nerves, meninges; and various parts of the central nervous system; connective, subcutaneous and other soft tissues; retroperitoneum, peritoneum; adrenal gland; thyroid gland; endocrine glands and related structures; female genital organs such as ovary, uterus, cervix uteri; corpus uteri, vagina, vulva; male genital organs such as penis, testis and prostate gland.

The cancer may be selected from the group consisting of: glioblastoma, lung cancer, non-small cell lung cancer (NSCLC), ovarian cancer, bladder cancer, rectal cancer, cervical cancer, and head and neck cancer.

The cancer may be selected from the group consisting of: benign, metastatic and malignant neoplasias, and also including acral lentiginous melanoma, actinic keratoses, adenocarcinoma, adenoid cycstic carcinoma, adenomas, adenosarcoma, adenosquamous carcinoma, astrocytic tumors, Bartholin gland carcinoma, basal cell carcinoma, bronchial gland carcinomas, capillary, carcinoids, carcinoma, carcinosarcoma, cavernous, cholangiocarcinoma, chondosarcoma, choriod plexus papilloma/carcinoma, clear cell carcinoma, cystadenoma, endodermal sinus tumor, endometrial hyperplasia, endometrial stromal sarcoma, endometrioid adenocarcinoma, ependymal, epitheloid, Ewing's sarcoma, fibrolamellar, focal nodular hyperplasia, gastrinoma, germ cell tumors, glioblastoma, glucagonoma, hemangiblastomas, hemangioendo-thelioma, hemangiomas, hepatic adenoma, hepatic adenomatosis, hepatocellular carcinoma, insulinoma, intaepithelial neoplasia, interepithelial squamous cell neoplasia, invasive squamous cell carcinoma, large cell carcinoma, leiomyosarcoma, lentigo maligna melanomas, malignant melanoma, malignant mesothelial tumors, medulloblastoma, medulloepithelioma, melanoma, meningeal, mesothelial, metastatic carcinoma, mucoepidermoid carcinoma, neuroblastoma. neuroepithelial adenocarcinoma nodular melanoma, oat cell carcinoma, oligodendroglial, osteosarcoma, pancreatic polypeptide. papillary serous adenocarcinoma, pineal cell, pituitary tumors, plasmacytoma, pseudosarcoma, pulmonary blastoma, renal cell carcinoma, retinoblastoma, rhabdomyosarcoma, sarcoma, serous carcinoma, small cell carcinoma, soft tissue carcinomas, somatostatin-secreting tumor, squamous carcinoma, squamous cell carcinoma, submesothelial, superficial spreading melanoma, undifferentiated carcinoma, uveal melanoma, verrucous carcinoma, vipoma, well differentiated carcinoma. and Wilm's tumor.

In some embodiments, the minibeam generated through the method according to the invention is used for minibeam radiation therapy in combination with any anticancer drug.

In some embodiments, the MSN according to the invention may be used for the treatment of cancer in combination with any anticancer drug.

In some embodiments, the MSN according to the invention may be used in combination with additional cancer therapies. In particular, the MSN according to the invention may be used in combination with targeted therapy, immunotherapy such as immune checkpoint therapy and immune checkpoint inhibitor, co-stimulatory antibodies, or chemotherapy.

Immune checkpoint therapy such as checkpoint inhibitors include, but are not limited to programmed death-1 (PD-1) inhibitors, programmed death ligand-1 (PD-L1) inhibitors, programmed death ligand-2 (PD-L2) inhibitors, lymphocyte-activation gene 3 (LAG3) inhibitors, T-cell immunoglobulin and mucin-domain containing protein 3 (TIM-3) inhibitors, T cell immunoreceptor with Ig and ITIM domains (TIGIT) inhibitors, B- and T-lymphocyte attenuator (BTLA) inhibitors, V-domain Ig suppressor of T-cell activation (VISTA) inhibitors, cytotoxic T-lymphocyte-associated protein 4 (CTLA4) inhibitors, Indoleamine 2,3-dioxygenase (IDO) inhibitors, killer immunoglobulin-like receptors (KIR) inhibitors, KIR2L3 inhibitors, KIR3DL2 inhibitors and carcinoembryonic antigen-related cell adhesion molecule 1 (CEACAM-1) inhibitors. In particular, checkpoint inhibitors include antibodies anti-PD1, anti-PD-L1, anti-CTLA-4, anti-TIM-3, anti-LAG3. Co-stimulatory antibodies deliver positive signals through immune-regulatory receptors including but not limited to ICOS, CD137, CD27, OX-40 and GITR.

Example of anti-PD1 antibodies include, but are not limited to, nivolumab, cemiplimab (REGN2810 or REGN-2810), tislelizumab (BGB-A317), tislelizumab, spartalizumab (PDR001 or PDR-001), ABBV-181, JNJ-63723283, BI 754091, MAG012, TSR-042, AGEN2034, pidilizumab, nivolumab (ONO-4538, BMS-936558, MDX1106, GTPL7335 or Opdivo), pembrolizumab (MK-3475, MK03475, lambrolizumab, SCH-900475 or Keytruda) and antibodies described in International patent applications WO2004004771, WO2004056875, WO2006121168, WO2008156712, WO2009014708, WO2009114335, WO2013043569 and WO2014047350.

Example of anti-PD-L1 antibodies include, but are not limited to, LY3300054, atezolizumab, durvalumab and avelumab.

Example of anti-CTLA-4 antibodies include, but are not limited to, ipilimumab (see, *e*.*g*., US patents US6,984,720 and US8,017,114), tremelimumab (see, *e*.*g*., US patents US7,109,003 and US8,143,379), single chain anti-CTLA4 antibodies (see, *e*.*g*., International patent applications WO1997020574 and WO2007123737) and antibodies described in US patent US8,491,895.

Example of anti-VISTA antibodies are described in US patent application US20130177557.

Example of inhibitors of the LAG3 receptor are described in US patent US5,773,578.

Example of KIR inhibitor is IPH4102 targeting KIR3DL2.

In some embodiments, the MSN according to the invention may be used in combination with targeted therapy. Targeted therapy agents, are drugs designed to interfere with specific molecules necessary for tumor growth and progression. For example, targeted therapy agents such as therapeutic monoclonal antibodies target specific antigens found on the cell surface, such as transmembrane receptors or extracellular growth factors. Small molecules can penetrate the cell membrane to interact with targets inside a cell. Small molecules are usually designed to interfere with the enzymatic activity of the target protein such as for example proteasome inhibitor, tyrosine kinase or cyclin-dependent kinase inhibitor, histone deacetylase inhibitor. Targeted therapy may also use cytokines. Examples of such targeted therapy include with no limitations: Ado-trastuzumab emtansine (HER2), Afatinib (EGFR (HER1/ERBB1), HER2), Aldesleukin (Proleukin), alectinib (ALK), Alemtuzumab (CD52), axitinib (kit, PDGFRbeta, VEGFR1/2/3), Belimumab (BAFF), Belinostat (HDAC), Bevacizumab (VEGF ligand), Blinatumomab (CD19/CD3), bortezomib (proteasome), Brentuximab vedotin (CD30), bosutinib (ABL), brigatinib (ALK), cabozantinib (FLT3, KIT, MET, RET, VEGFR2), Canakinumab (IL-1 beta), carfilzomib (proteasome), ceritinib (ALK), Cetuximab (EGFR), cofimetinib (MEK), Crizotinib (ALK, MET, ROS1), Dabrafenib (BRAF), Daratumumab (CD38), Dasatinib (ABL), Denosumab (RANKL), Dinutuximab (B4GALNT1 (GD2)), Elotuzumab (SLAMF7), Enasidenib (IDH2), Erlotinib (EGFR), Everolimus (mTOR), Gefitinib (EGFR), Ibritumomab tiuxetan (CD20), Sonidegib (Smoothened), Sipuleucel-T, Siltuximab (IL-6), Sorafenib (VEGFR, PDGFR, KIT, RAF),(Tocilizumab (IL-6R), Temsirolimus (mTOR), Tofacitinib (JAK3), Trametinib (MEK), Tositumomab (CD20), Trastuzumab (HER2), Vandetanib (EGFR), Vemurafenib (BRAF), Venetoclax (BCL2), Vismodegib (PTCH, Smoothened), Vorinostat (HDAC), Zivaflibercept (PIGF, VEGFA/B), Olaparib (PARP inhibitor).

In some embodiments, the MSN according to the invention may be used in combination with chemotherapy. As used herein, the term "chemotherapy" has its general meaning in the art and refers to the treatment that consists in administering to the patient a chemotherapeutic agent. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g. , calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; methylhydrazine derivatives including N-methylhydrazine (MIH) and procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; anthracyclines, nitrosoureas, antimetabolites, epipodophylotoxins, enzymes such as L-asparaginase; anthracenediones; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide; and pharmaceutically acceptable salts, acids or derivatives of any of the above.

The invention is not restricted to embodiments described above and numerous adjustments may be made within the scope of the invention.

Thus, in combinable alternatives of previous embodiments:
- the SDD 1, and the support 3 according to the present embodiment, are arranged to tilt the collimator 2 relative to a point comprised in the single slit 4, preferably to a point of the central plane 5 of the slit 4.

## Claims

1. Method for producing a minibeam comprising the steps of:
- providing an incident beam (15) in the form of a scanning beam, and
- moving a single slit (4) of a collimator (2), the step consisting of moving the single slit of the collimator comprises:
• translating the collimator, by means of a scanning dynamic device (SDD) (1), in a plane (XY) perpendicular to a plane (XZ) whereby the single slit extends and/or in a direction parallel to the plane (XZ) whereby the single slit extends so that, at least temporarily and at least in part, the scanning beam goes through the single slit, and/or
• rotating the collimator, by means of the SDD, relative to a rotation axis parallel to the plane (XZ) whereby the single slit extends and
• tilting the collimator, by means of the SDD, relative to a predefined reference axis (10) and/or relative to a point comprised in the single slit so that the scanning beam remains parallel to a plane (XZ) whereby the single slit extends.

2. Method according to the previous claim, whereby the step of moving the collimator (2) comprises the steps of:
- positioning the single slit (4) so that a central plane (5) of the single slit, which extends along the plane (XZ) whereby the single slit extends and comprises a length (8) of the single slit, is aligned with a scan direction of the scanning beam (15),
- when the scanning beam reaches a center of the single slit, translating the single slit according to the scan direction so that the scanning beam remains at the center of the single slit.

3. Method according to claim 13, whereby the step of moving the collimator comprises the steps of:
- positioning the single slit (4) so that the scanning beam (15), when emission of the scanning beam is started, goes through the single slit at a longitudinal end of the single slit,
- positioning the single slit so that a non-zero angle is formed between the length of the single slit and a scan direction of the scanning beam,
- emitting the scanning beam and moves the scanning beam according to the scan direction,
- translating the single slit in a direction perpendicular to the plane (XZ) whereby the single slit extends so that the scanning beam goes through the single slit,
- when the scanning beam reaches the other longitudinal end of the single slit, stopping the scanning beam emission.
